# EUROPÄISCHE PATENTSCHRIFT

(11) **EP 1 060 035 B1**
(45) Veröffentlichungstag und Bekanntmachung des Hinweises auf die Patenterteilung: **04.06.2003**
(21) Anmeldenummer: 99915545.0
(22) Anmeldetag: 05.03.1999
(51) Int. Cl.: B09B 3/00, B03B 9/06, C10J 3/00, C02F 3/28

(54) **VERFAHREN UND VORRICHTUNG ZUR AUFBEREITUNG VON RESTSTOFFGEMENGEN UND ZUR KONVERSION VON KOHLENSTOFFHALTIGEN REST- ODER ROHSTOFFEN IN DEN RESTSTOFFGEMENGEN**
METHOD AND DEVICE FOR PROCESSING REFUSE MATERIAL BATCHES AND FOR CONVERTING CARBON-CONTAINING REFUSE OR RAW MATERIALS INTO REFUSE MATERIAL BATCHES
PROCEDE ET DISPOSITIF POUR TRAITER DES MELANGES DE MATIERES RESIDUELLES ET POUR CONVERTIR DES MATIERES RESIDUELLES OU BRUTES PRESENTANT UNE CERTAINE TENEUR EN CARBONE, CONTENUES DANS LESDITS MELANGES DE MATIERES RESIDUELLES

(30) Priorität: 05.03.1998 DE 19809400
(43) Veröffentlichungstag der Anmeldung: 20.12.2000
(73) Patentinhaber: Staiger, Roland, 81675 München (DE); Staiger, Petra, 82041 Oberhaching (DE)
(72) Erfinder: HIRSCHMANN, Günter, D-85667 Oberpframmern (DE)
(74) Vertreter: Niederkofler, Oswald A., Dipl.-Phys.
(86) Internationale Anmeldenummer: EP9901439
(87) Internationale Veröffentlichungsnummer: WO99044761

(56) Entgegenhaltungen:
- EP-A- 0 359 250
- EP-A- 0 607 644
- WO-A-97/41193
- US-A- 3 687 646
- US-A- 4 289 625
- PATENT ABSTRACTS OF JAPAN vol. 14, no. 197 (C-712), 23.April 1990 & JP 02 040300 A (TADAYOSHI), 9.Februar 1990,

## Beschreibung

Die vorliegende Erfindung betrifft Verfahren zur Aufbereitung von Reststoffgemengen und zur Konversion von kohlenstoff-haltigen Rest- oder Rohstoffen in den Reststoffgemengen sowie eine Vorrichtung zur Durchführung derartiger Verfahren.

Aus der Literatur sind zahlreiche Verfahren zur Mülloder Reststoffaufbereitung bzw. zur Verwertung der in organischem Müll gespeicherten Energie bekannt. In der DE 44 02 559 C2 wird bspw. ein Verfahren bzw. eine Vorrichtung zur Konversion pflanzlich gebundener Sonnenenergie und von biologischem Material offenbart. Hierbei werden verschiedene Verfahrenslinien beschrieben, die abhängig vom biologischen Ausgangsmaterial spezifisch die Umwandlung zu wiederverwendbaren Endprodukten gestatten. Insbesondere ist auch eine Vergärungsstufe mit Alkoholdestillation des organischen Materialbreis vorgesehen. Der während der Vergärungsstufe anfallende Alkohol wird nach der Lehre der DE 44 02 559 C2 von den übrigen Vergärungsprodukten abgetrennt, wobei die übrigen Vergärungsprodukte, z.B. Gas, Fuselöle und Schlempe, einem Blockheizkraftwerk zugeführt werden.

Aus der US-A-4,289,625 ist ein Verfahren zur Aufbereitung von Reststoffmengen (Müll) und zur Konversion von kohlenstoffhaltigen Rest- und Rohstoffgemengen bekannt, bei dem a) die in den Restoffgemengen (Stadtmüll) enthaltenen kohlenstoffhaltigen Rest- und Rohstoffe (Speisereste) von anorganischen und metallischen Materialien getrennt werden, b) Flüssigrohmaterial in einem Reaktor einer Fermentation unterzogen wird und c) das im Zuge der Fermentation gebildete Biogas in mindestens einem Kraftwerk zur Energiegewinnung eingesetzt wird. Von diesem Verfahren unterscheidet sich das erfindungsgemäße Verfahren durch eine Vorentwässerung der kohlenstoffhaltigen Rest- und Rohstoffe, die Aufgabe der flüssigen Bestandteile in die Fermentation und die Pelletierung der entwässerten Stoffe, die dann nur dem Druckreaktor aufgegeben werden.

Die WO 97/41193 betrifft ein Verfahren und eine Vorrichtung zum Verarbeiten von biologischen Reststoffen, insbesondere Kläschlamm, in dem entwässerter Klärschlamm getrocknet wird, dann in einem Wirbelschichtvergaser unter Sauerstoffmangel vergast wird und das Produktgas in einem Blockkraftwerk verwendet wird. Eine direkte Aufarbeitung von Reststoffgemengen (Müll) ist nicht erwähnt.

Zwar unterliegt das in der DE 44 02 559 C2 vorgeschlagene Verfahren einer, strikten Beachtung des Kreislaufprinzips, doch weisen die dort offenbarten Verfahren den Nachteil auf, daß nur ein suboptimaler Wirkungsgrad, bezogen auf die in den Rest- und Abfallstoffen gespeicherte chemische Energie, erzielt wird. Auch ist das Problem der vom Blockheizkraftwerk ausgestoßenen Abgase nicht befriedigend gelöst. Ferner sind stets mehrere Verfahrenslinien zur Behandlung verschiedener organischer Ausgangsstoffe erforderlich.

Insofern ist es die Aufgabe der vorliegenden Erfindung, unter strenger Berücksichtigung des Kreislaufprinzips den Wirkungsgrad bei der Verwertung kohlenstoffhaltiger Restund/oder Rohstoffe zu verbessern, die sich aus der Verfahrensdurchführung ergebende ökologische Belastung weitestgehend zu vermindern und zugleich ein einfaches, vom organischen Ausgangsmaterial unabhängiges Verfahren zur Verfügung zu stellen.

Die Lösung dieser Aufgabe erfolgt durch ein Verfahren nach Anspruch 1 bzw. durch eine Vorrichtung nach Anspruch 13.

Erfindungsgemäß wird ein Verfahren offenbart, das zur Aufbereitung von Reststoffgemengen und zur Konversion von kohlenstoffhaltigen Rest- oder Rohstoffen in den vorgenannten Reststoffgemengen
(a) eine Trennung der in den Reststoffgemengen enthaltenen kohlenstoffhaltigen Rest- oder Rohstoffen von anorganischen und/oder metallischen Feststoffen,
(b) eine Entwässerung der abgetrennten kohlenstoffhaltigen organischen Rest- oder Rohstoffe und deren Pelletierung,
(c) eine Fermentation der im Rahmen der Entwässerung erhaltenen Abpreßwässer in einem oder mehreren Reaktoren,
(d1) eine Weiterleitung der im Rahmen der Fermentation nach Verfahrensschritt (c) gebildeten Biogase an mindestens ein Kraftwerk und/oder
(d2) Zufuhr des Biogases zu mindestens einem Druckreaktor und Behandlung des Biogases in demselben zusammen mit den gemäß (b) pelletierten Rest- oder Rohstoffen, die darin aufgeschlossen bzw. vergast werden,
vorsieht.

Das erfindungsgemäße Verfahren trägt wesentlich zur Entlastung der Umwelt und zur Verbesserung der Energiebedarfssituation bei. Als Feststoffgemenge kommt jede Kombination von organischem und anorganischem Abfall in Frage. Der aufzubereitende organische Abfall umfaßt insbesondere Abfallprodukte aus der Landwirtschaft wie auch aus der Lebensmittelindustrie. Aus der Landwirtschaft eignen sich ganz besonders die Gülle von Schweinen, Rindern, Geflügel oder anderen landwirtschaftlichen Nutztieren, aus der Lebensmittelindustrie dagegen die Endprodukte aus Schlachtereien, fischverarbeitenden Betrieben, Gaststätten, Mühlen oder Bäckereien. Weiterhin können nachwachsende Rohstoffe, z.B. Stroh oder Heu, Holzabfälle, aus der Landwirtschaft oder auch aus der holzverarbeitenden Industrie, insbesondere Putz- oder Altholz, Abscheider- und Frittierfette sowie organische Materialien anderer Quellen, als organische Abfallstoffe zur Verarbeitung gelangen.

Nach Anlieferung der Abfälle erfolgt die Abtrennung der Eisenteile und die Entfernung anderer anorganischer, insbesondere mineralischer, oder metallischer Störstoffe. Erfindungsgemäß wird somit der Aufbereitung und Verwertung der organischen Abfallstoffe eine Trennstufe vorgeschaltet, die die Grobtrennung anorganischer und organischer Reststoffe aus dem Gemenge erlaubt.

Vorzugsweise wird der nach der Trennstufe im wesentlichen organische Abfall auf einen oder mehrere Schredder gegeben. Der (die) Schredder hat (haben) die Aufgabe, die Abfall auf Korngröße zu zerkleinern. Typischerweise sollte eine Zerkleinerung auf eine Korngröße des Abfalls von weniger als 500 mm, vorzugsweise weniger als 300 mm, insbesondere weniger als 250 mm, erreicht werden. Durch die Schredderleistung wird gleichzeitig auch eine Durchmischung der Restabfälle bewirkt. Auch vermag der Zerkleinerungsschritt ausreichend große Oberflächen für den ersten Windsichtungs- und Siebungsprozeß, der vorzugsweise der Entwässerung vorgeschaltet ist, zu gewährleisten. In einer besonders bevorzugten Ausführungsform des erfindungsgemäßen Verfahrens werden die Schredder- und Siebungsschritte zwei- oder mehrfach wiederholt.

Erfindungsgemäß wird der Abfall nunmehr mit Hilfe einer Siebpresse entwässert. Das Abpreßwasser wird einem Fermenter zugeführt. In einer bevorzugten Ausgestaltung kann jedoch ein gewisser Anteil des Abpreßwassers auch an einen oder mehrere Druckreaktor(en) weitergeleitet werden. Die nach der Entwässerung erhaltene Festfraktion kann danach vorzugsweise in weiteren Verfahrensschritten aufbereitet und schließlich nach der Pelletierung in Form von Pellets in einen Druckreaktor(en) eingeführt werden. Die vorzugsweise der Pelletierung vorangehenden Sichtungs-, Siebungs- und Fraktionierungsschritte stellen dabei sicher, daß die Pellets eine chemische und physikalische Homogenität aufweisen, die den Aufschluß bzw. die Vergasung der Abfallstoffe im (in den) Druckreaktor(en) weiter verbessern können.

Die abgetrennten anorganischen oder metallischen Inertstoffe können indes einer anderweitigen Verwertung zugeführt werden. So etwa können die abgetrennten metallischen Bestandteile als Altmetall, die mineralischen Bestandteile als Bauzuschlagstoffe im Straßenbau, für Drainagefüllungen oder bei Einbettungen für Rohrleitungen eingesetzt werden.

Das nach dem erfindungsgemäßen Verfahren im Zuge des Verfahrensschritts (b) erhaltene Abpreßwasser wird als Fermentersubstrat weiterverarbeitet. Hierzu wird es gemischt und zunächst in einen Fermentationsreaktor gepumpt. Die Fermentation in diesem Reaktor wird bei einer Temperatur oberhalb von 20°C, typischerweise bei Temperaturen zwischen 30 und 40°C, also im mesophilen Bereich, durchgeführt. Typischerweise beträgt die Verweilzeit des Abpreßwassersubstrats in dem Reaktor 5 bis 15 Tage, besonders bevorzugt zwischen 8 und 10 Tagen. In einer bevorzugten Ausgestaltung des Fermentationsverfahrens wird das Fermentersubstrat in dem ersten Reaktor zunächst vor- oder teilvergoren und dann in einem oder mehreren weiteren Reaktor(en) weitervergoren. Ein zweiter Reaktor kann typischerweise bei einer höheren Temperatur als der erste Reaktor arbeiten, z.B. von 40 bis 60°C, bevorzugt von 50 bis 60°C, also im thermophilen Bereich. Das zunächst im ersten Reaktor vorvergorene Substrat wird dann in dem (den) folgenden Reaktor(en) weiter abgebaut. Derart kann eine Aneinanderreihung von Reaktoren mit jeweils steigender Reaktionstemperatur verfahrensgemäß vorteilhaft sein.

Die mittlere Verweilzeit des Substrats in einem gegebenenfalls verfahrensgemäß vorgesehenen zweiten oder weiteren Reaktor beträgt zwischen 1 und 15 Tagen, insbesondere bevorzugt zwischen 8 und 10 Tagen.

Das in dem Fermenter produzierte Biogas wird nach der Kondensatabscheidung entschwefelt. Die Entschwefelung wird über eine stöchiometrische Luftzugabe zum Biogas ermöglicht. Das vorzugsweise getrocknete und entschwefelte Biogas wird dann an ein Kraftwerk und/oder an einen Druckreaktor weitergeleitet.

In einem oder mehreren Kraftwerk(en), typischerweise ein oder mehrere Blockheizkraftwerke mit Gasmotor und/oder Gasturbine, wird das Biogas verbrannt. Der Heizwert des Biogases wird zur Gewinnung elektrischer Energie herangezogen, die den Eigenstrombedarf zur Durchführung des Verfahrens deckt.

Ein weiterer Teil des Biogases wird, wie oben beschrieben, in einen Druckreaktor geführt und dort gemeinsam mit den gegebenenfalls getrockneten Pellets behandelt. Vorzugsweise wird diese Behandlung mit Wasserdampf in einer stationären Wirbelschicht vorgenommen.

In einer vorteilhaften Ausführungsform wird das im Druckreaktor erzeugte Produktgas zunächst typischerweise durch einen Filter feinentstaubt und ggfs. entschwefelt. Das Produktgas weist einen hohen Wasserstoffanteil auf. Typischerweise liegt dieser Wasserstoffanteil bei über 50 Vol.-%. Das Produktgas kann damit direkt als Wertstoff, z.B. als Brennstoff oder Treibstoff, abhängig vom Reinigungs- und Aufbereitungsgrad, eingesetzt werden. In einer bevorzugten Ausführungsform des vorliegenden Verfahrens wird jedoch Wasserstoff aus dem Produktgasgemenge abgetrennt. Diese Abtrennung erfolgt vorteilhafterweise durch einen Gasfilter. Dabei kommen typischerweise Druckwechselabsorptions- oder Membrantrennverfahren in Frage, um den Wasserstoff, aufzukonzentrieren. Der derart angereicherte Wasserstoff kann nunmehr zur Stromerzeugung in einer oder mehreren Brennstoffzelle(n) eingesetzt werden.

Besonders bevorzugt ist der Einsatz des aufgereinigten Wasserstoffs zur Synthese von Methanol. Hierzu wird der Wasserstoff mit Abgasen, insbesondere solchen Abgasen, die einen hohen Kohlenmonoxydanteil aufweisen, umgesetzt. Erfindungsgemäß wird hierfür bevorzugt jener Abgasstrom eingesetzt, der gemäß dem erfindungsgemäßen Verfahren im Zuge der Verbrennungsreaktion im Kraftwerk entsteht. Diese Umsetzung führt zu einer vollständigen Entsorgung des im Fermentierungsreaktor produzierten Biogases. Das dem erfindungsgemäßen Verfahren zugrunde liegende Kreislaufsystem schließt somit auch die Entsorgung von im Verfahren etwaig anfallenden Kohlenmonoxyds ein.

Darüber hinaus kann überschüssiger, ggf. aufgereinigter Wasserstoff als Vergasungsprodukt in Wasserstofftanks zwischengelagert werden und erst im Bedarfsfall entweder zur Methanolsynthese oder zur Beschickung von Brennstoffzellen eingesetzt werden.

Das erfindungsgemäße Verfahren erlaubt es somit, die organischen Rest- oder Rohstoffe unter Optimierung des Wirkungsgrads zu Wasser, CO₂ und Methanol abzubauen. Die darüber hinaus in dem (den) Fermentationsreaktor(en), dem (den) Kraftwerk(en) und dem (den) Druckreaktor(en) entstehenden flüssigen und/oder festen Reststoffe finden ihre Verwendung als Flüssig- oder Festdünger in der Landwirtschaft. Ferner ergibt sich aus der Tatsache, daß das Verfahren mit nachwachsenden Roh- oder Reststoffen durchgeführt wird, eine in Hinblick auf die CO₂-Emissionen ausgeglichene und umweltneutrale Bilanz.

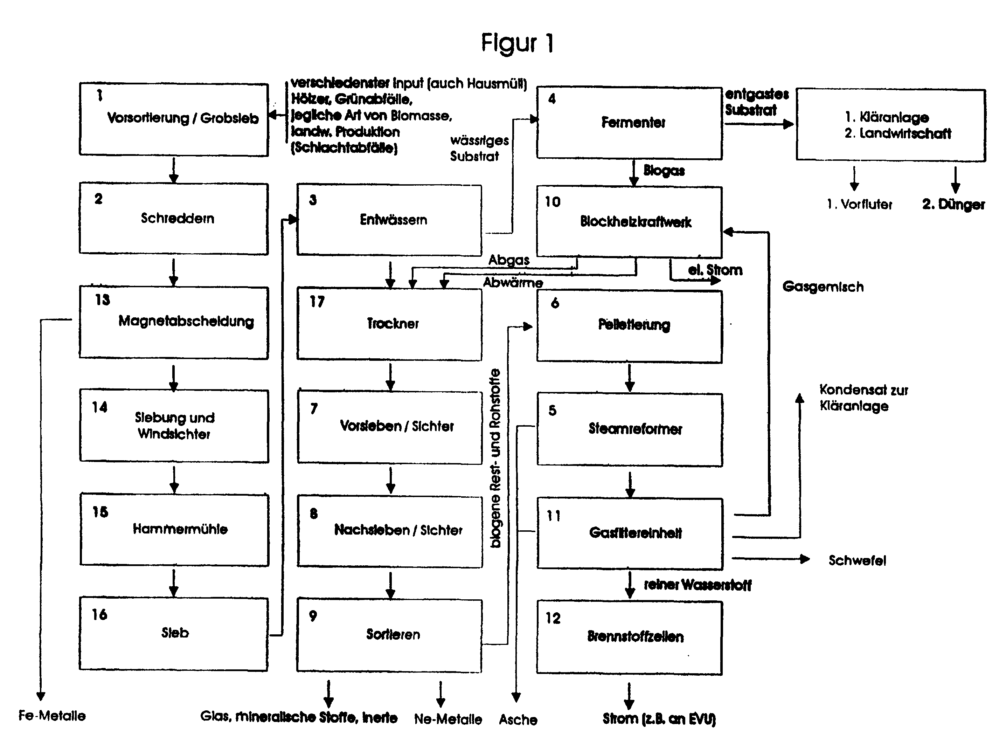

Weitere Einzelheiten, Merkmale und Vorteile der Erfindung ergeben sich aus nachfolgender Beschreibung eines Ausführungsbeispiels anhand der Zeichnung. Figur 1 zeigt schematisch ein vereinfachtes Blockschaltbild, wobei dieses Schaltbild sowohl ein erfindungsgemäßes Verfahrensschema als auch den Aufbau einer zur Durchführung eines erfindungsgemäßen Verfahrens geeigneten erfindungsgenäßen Vorrichtung widerspiegelt. Figur 1 stellt neben den Basiskomponenten des erfindungsgemäßen Verfahrens bzw. der erfindungsgemäßen Vorrichtung auch weitere Elemente als Bestandteile bevorzugter Ausführungsformen dar.

Das erfindungsgemäße Verfahren wird mit Hilfe einer gleichfalls erfindungsgemäßen vorrichtung durchgeführt, die einen oder mehrere Fermentationsreaktor(en) (4), einen oder mehrere Druckreaktor(en) (5) und ein oder mehrere Blockheizkraftwerk(e) (10) umfaßt. Gleichfalls umfaßt die erfindungsgemäße Vorrichtung Mittel zum Vorsortieren und Trennen des Abfallmaterials (1) in die Fraktionen anorganischer bzw. metallischer Konsistenz und organischer kohlenstoffhaltiger Konsistenz. Darüber hinaus unfaßt die erfindungsgemäße Vorrichtung Mittel zum Entwässern und Pelletieren (3) der organischen Rest- oder Rohstoffkomponenten. Diese Elemente der erfindungsgemäßen Vorrichtung stellen die Grundausrüstung zur Durchführung des erfindungsgemäßen Verfahrens dar. Die vorgenannten Basiselemente der erfindungsgemäßen Vorrichtung können um weitere optionale Elemente ergänzt werden.

Vorzugsweise werden die aufzubereitenden Abfallstoffe auf einer Förderschnecke vom zentralen Sammelbunker in die erfindungsgemäße Vorrichtung transportiert. Ein Magnetförderband mit Mitteln zum Vorsortieren (1), bspw. mit einem oder mehreren Abscheiderechen und einem oder mehreren Walzenmagnet(en), dient zur Trennung von Metallteilen und zur Entfernung grober Störstoffe. Insbesondere sind hier Steine, Glas, Keramik und beliebige Metallbestandteile zu nennen. Dieses, insbesondere nicht-organische Schwergut wird dann in einer optionalen Ausgestaltung des erfindungsgemäßen Verfahrens bzw. der erfindungsgemäßen Vorrichtung über ein Transportband abgeführt und schließlich über Eisen- bzw. Nicht-Eisenabscheider (13) getrennt. Der optionale Eisenabscheider kann als Trommelmagnet ausgebildet sein , als Nicht-Eisenabscheider kommen vorzugsweise Wirbelstromabscheider zum Einsatz.

Die durch Mittel zum Vorsortieren (1), beispielsweise Abscheiderechen und Walzenmagnet(en), auf dem Magnetförderband nicht abgetrennten, insbesondere organischen Abfallbestandteile werden einem Schredder (2) zugeführt, der ein Aufreißer, und Zerkleinern dieser Hauptfraktion bewirkt. Die als Folge des Zerkleinerungsvorgangs anfallenden Partikel sollten eine Korngröße von weniger als 500 mm, vorzugsweise von weniger als 300 mm, ganz besonders bevorzugt von weniger als 250 mm, haben. Dieser Zerkleinerungsvorgang bewirkt außerdem eine intensive Durchmischung der Bestandteile der Hauptfraktion.

Als weiteres optionales Element kann die erfindungsgemäße Vorrichtung auch Mittel zum Windsichten (14) aufweisen. Durch eine derartige Windsichtung wird von der Hauptfraktion eine Leichtfraktion, die im allgemeinen z.B. Papier oder Kunststoffolien enthält, abgetrennt. Im Anschluß daran erfolgt typischerweise eine Siebung der Hauptfraktion in einem Grobsieb (14), wobei der Siebschnitt entsprechend dem Grad der zuvor erreichten Zerkleinerung, also der Korngröße, gewählt wird. Der Siebschnitt dürfte typischerweise zwischen 250 und 500 mm liegen. Die zerkleinerte und gesiebte Hauptfraktion mit Korngrößen von beispielsweise weniger als 250 mm kann vorzugsweise abermals einem Schredder, z. B. einer Hammermühle (15) zur Zerkleinerung zugeführt werden. Diese Zerkleinerung sollte in der Hauptfraktion Körner mit Korngrößen, die kleiner als jene nach dem ersten Zerkleinerungs- bzw. Siebungsschritts sind, also z.B. zu Korngrößen von weniger als 100 mm, ergeben. Vorzugsweise sollten die Körner nach der Zerkleinerung eine Maximalgröße von weniger als 60 mm aufweisen. Diesem optionalen Zerkleinerungsschritt folgt vorzugsweise ein weiterer Siebungsschritt in einem Sieb (16) mit einem adäquaten siebschnitt. Typischerweise liegt der Siebschnitt bei diesem Siebungsschritt zwischen 60 und 100 mm, vorzugsweise bei ca. 60 mm. Die einzelnen Zerkleinerungs- und Siebungsschritte können jeweils für die im Sieb verbliebene Fraktion wiederholt werden, um einen Großteil der Festfraktion mit entsprechender Korngröße der Weiterverarbeitung in der erfindungsgemäßen Vorrichtung zuführen zu können.

Als Basiselement zur Durchführung des erfindungsgenäßen Verfahrens folgt nach der zuvor beschriebenen optionalen Vorbehandlung der organischen Hauptfraktion zu Körnern das Abpressen derselben in Mitteln zum Entwässern, z. B. einer Siebpresse (3). Die Entwässerung der Hauptfraktion in der Siebpresse (3) bewirkt eine Auftrennung in eine flüssige Fraktion unc eine Festfraktion, die auf unterschiedlichen Wegen in der erfindungsgemäßen Vorrichtung weiterbearbeitet werden können.

Die flüssige Fraktion wird entweder einem oder mehreren Fermentierungsreaktor(en) (4) und/oder einem oder mehreren Druckreaktor(en) (5) zugeführt. Vorzugsweise wird der Hauptteil der Flüssigfraktion in einem oder mehreren Fermentierungsreaktor(en) (4) auf biologischem Weg abgebaut.

Die Festfraktion wird erfindungsgemäß für die Vergasung in einem oder mehreren Druckreaktor(en) (5) vorbereitet. Hierzu ist erfindungsgemäß eine Pelletierung in einer Pelletiervorrichtung (6) erforderlich. Vorzugsweise wird jedoch vor dem Pelletierungsschritt die Festfraktion zunächst einer Trocknung unterzogen. Die Trocknungsvorrichtung (17) kann hierbei als Bandtrockner ausgestaltet sein und ein Bandtrocknerband, Vorlagebehälter, Verteil- und Sammelschnecke(n) und Wärmeaustauscher umfassen. In einer besonders bevorzugten erfindungsgemäßen Ausgestaltungsform werden vor dem Pelletierungsschritt im Pelletierer jeweils einzeln oder in beliebiger Kombination Zerkleinerungs-, Siebungs-, Windsichtungsschritte und/oder Sortierungsschritte in Mitteln zum Zerkleinern, Sieben, Windsichten (7 und 8) und zum Sortieren (9) durchgeführt.

Hierfür kann die gegebenenfalls getrocknete Festfraktion auf einem geschlossenen Transportband in einen Bunker gefördert werden. Als besonders vorteilhaft erweist sich eine Beschickung eines oder mehrerer Trommelsiebe/s mit der körnigen Festfraktion zur Absiebung von grobkörnigen Teilchen, z.B. mit einer Größe von über 60 mm, vorzugsweise von über 40 mm. Der Siebschnitt des Trommelsiebs wird entsprechend gewählt. Das Verfahren kann dabei ganz besonders bevorzugt auch so ausgeführt werden, daß die jeweils im Sieb verbliebene Fraktion nochmals zerkleinert und dann abermals gesiebt wird. Derartige Schritte können auch mehrfach zyklisch wiederholt werden. Als Ergebnis dieser Kombination von Siebung, vorzugsweise einer Trommelsiebung, Zerkleinerung, Windsichtung und/oder Sortierung liegt eine homogene Festfraktion mit einer einheitlichen Körnung, z.B. mit einer Korngröße von weniger als 40 mm, vor.

In einer ganz besonders bevorzugten Ausführungsform der vorliegenden Erfindung wird das körnige Material in einer anschließenden weiteren Siebung in eine feinkörnige und grobkörnige Fraktion aufgetrennt werden. So etwa kann die Trennung in fein- und grobkörnige Festfraktion bspw. bei einem Siebschnitt von 10 bis 20 mm, vorzugsweise bei 15 mm, durchgeführt werden. Die klein- und die grobkörnige Festfraktion können dann, voneinander getrennt, einer trockenen Dichtesortierung zugeführt werden, um die Trennschärfe des Sortierverfahrens zu gewährleisten.

Zur Vorbehandlung der Festfraktion ist auch der Einsatz eines oder mehrerer Windsichter(s) (7 bzw. 8) bevorzugt. Der Windsichter (7 bzw. 8) erlaubt die Trennung des organischen Materials von etwaigen verbliebenen Inertstoffen, insbesondere mineralischen Komponenten, wobei die nach der Windsichtung erhaltene Leichtfraktion zu Pellets weiterverarbeitet wird, während die Schwerfraktion aus im wesentlichen Inertstoffen als Nebenprodukt ausgeschieden wird. Um den Dauerbetrieb der erfindungsgemäßen Vorrichtung sicherzustellen, können vor und/oder nach dem Pelletierungsschritt Vorratsbehälter als Speicher eingerichtet werden. Auf diese Weise gelingt es, beim Betrieb der Vorrichtung ein kinetisches Gleichgewicht ("steady state") dauerhaft aufrechtzuerhalten.

Es folgt erfindungsgemäß der Pelletierungsschritt im Pelletierer (6). Die Pellets dienen als Brennstoff für den nachfolgenden Vergasungsschritt im Druckreaktor (5). Sie weisen eine chemische und physikalische Homogenität auf, die einen gleichmäßigen Betrieb des Druckreaktors (5) gestattet. Die üblicherweise im Abfall auftretenden Schwankungen der Inhaltstoffe sind durch das beschriebene Sortierverfahren deutlich reduziert. Durch die Fraktionierung der zur Beschickung der Anlage gelangenden Abfallstoffe in solche organischer und anorganischer Konsistenz, die ggf. erfolgende Vorbehandlung und die nachfolgende Pelletierung der Festfraktion kann erfindungsgemäß auf die nach dem Stand der Technik erforderlichen, verschiedener. Verarbeitungslinien, die - in Abhängigkeit von der Zusammensetzung und Konsistenz der zu verarbeitenden Abfallmaterialien - in einer Vorrichtung zur Abfallaufarbeitung zugeschaltet oder abgeschaltet werden müssen, verzichtet werden.

Eine Vergasungsvorrichtung wird nunmehr mit dem gegebenenfalls getrockneten, in Pelletform vorliegenden Rest- oder Rohstoff beschickt. Die Mittel zur Beschickung können ein Fördersystem, ggf. mit Aufsatzfilter(n), Schleusenbehälter, Schüttgutklappen und/oder Dosierschnecken umfassen.

Die nachfolgende Vergasungsreaktion wird bei erhöhten Drücken vorgenonunen, weswegen ein Druckreaktor (5) zur Durchführung des erfindungsgemäßen Verfahrens erforderlich ist. Vorteilhafterweise wird zum kohlenstoffhaltigen pelletförmigen Brennstoff Wasserdampf geführt. In diesem bevorzugten Fall wird als Druckreaktor (5) ein Wasserdampfdruckreaktor eingesetzt. Die in dem Reaktor erfolgende chemische Reaktion führt unter Oxidation des in den Pellets gebundenen Kohlenstoffs zu Gasgemischen, die im wesentlichen aus Wasserstoff, zu geringeren Teilen auch Kohlenmonoxyd und Kohlendioxyd und geringe Mengen anderer gasförmiger Produkte enthalten. Vorteilhafterweise beträgt der Anteil des Wasserstoffs in diesem Gasgemisch mehr als 50%.

Nach einer vorteilhaften Ausführungsform des erfindungsgemäßen Verfahrens bzw. der erfindungsgemäßen Vorrichtung ist der Vergaser derart ausgestaltet, daß die Vergasungsreaktion auf der Basis eines Wirbelschichtverfahrens ausgeführt wird. Dabei liegen die pelletförmigen Biomassen in einer stationären Wirbelschicht im Druckreaktor vor. Bei Anwendung eines Wirbelschichtverfahrens werden die Pellets über ein Schleusensystem, vorzugsweise eine Zellradschleuse, in den unter Überdruck stehenden Vergasungsgenerator eingebracht. In der Folge verteilen sich die Pellets auf waagrechten perforierten Böden und werden von unten von Wasserdampf durchströmt. Unter den entsprechenden Strömungsbedingungen stellt sich ein Zustand ein, bei dem die Teilchen in einer ständigen Auf- und Abbewegung wirbeln und sich daher in der Schwebe befinden. Die Teilchen werden in diesem Fall fluidisiert, d.h., sie befinden sich in einem Fluidat(oder Wirbel)bett.

Der wirbelschichtvergaser kann einen Wirbelbett-Gasgenerator, Druckbehälter des Gasgenerators, Dosierungsstutzen, Ascheaustrag, Wirbelbettrost, mehrschichtige Ausmauerung, Bettmaterialausschleusung mit druckdichten Abspertorganen, Sieb zur Klassierung des ausgeschleusten Bettmaterials der ausgetragenen Asche aus Wirbelschichtüberlauf und/oder Zyklonabscheider in drei Fraktionen umfassen.

Der fluidisierende Wasserdampf ist in einer besonders vorteilhaften Ausführungsform der vorliegenden Erfindung zugleich auch wärmeträgermedium. In diesem Fall kann auf alle wärmeübertragenden Einbauten im Vergaser verzichtet werden. Insofern ist im vorliegenden Fall ein allothermes Verfahren zur Vergasung von Biomasse bevorzugt.

Im Gegensatz zum autothermen Verfahren wird beim allothermen Verfahren ein externer Träger der Wärme, typischerweise Wasserdampf, an den zu vergasenden Stoff übertragen. Durch die vorzugsweise allotherme Verfahrensführung ist es nicht erforderlich, einen Teil der Vergasungsprodukte selbst zur Wärmeerzeugung zu verbrennen. Gleichfalls erlaubt das allotherme Verfahren die Produktion eines heizwertreichen Gases mit hohem Wasserstoffanteil, geringer staubfracht und einem geringen Feststoffcehalt, z.B. Teergehalt. Erfindungsgemäß kann jedoch die Vergasung auch autotherm erfolgen, wobei dann typischerweise ein Teil der Vergasungsprodukte im Generator mit Luft verbrannt wird.

Der vorzugsweise zur Vergasung verwendete Wasserdampf kann sich aus einer oder beliebigen Kombinationen der folgenden drei Quellen speisen: extern zugeführtes Brauchwasser und/oder das Verbrennungsprodukt eines Teils des wasserstoffreichen Produktgases und/oder Kondensat des im Abhitzekessel für die Abkühlung des Produktgases entstehenden Wassers. Die Mittel zur Erzeugung des Wasserdampfs können Rauchrohrdampfkessel, Speisewasservorwärmer, Speisewasserpumpen, Rohrleitungen und/oder Druckbrennkammer umfassen.

Vorteilhafterweise werden die gasförmigen Produkte aus dem ggf. im Wirbelschichtverfahren betriebenen Vergaser in einen oder mehrere Zyklon(e) überführt. Der zylindrische Zyklon mit konisch zulaufendem Boden erlaubt das Abscheiden der gröberen Staubteilchen durch die Zentrifugalkraft des tangential eintretenden Gas-/Staubstroms. Der vom Grobstaub befreite Gasstrom verläßt den Zyklon oben durch ein Austrittsrohr. Gegebenenfalls gelangt das Gas in einen Abhitzekessel und wird danach typischerweise durch die nach dem Stand der Technik bekannten Verfahren feinentstaubt. Hierzu eignen sich Gasreinigungs- und/oder Staubfilter. Der vorzugsweise feinentstaubte Gasstrom wird als wasserstoffreiches Gas entweder mit optionalen Elementen weiterbehandelt oder aber er wird vorteilhafterweise zu einem gewissen Teil, vorzugsweise zu weniger als 50%, nach Oxidation zu Wasserdampf für die Wirbelstromerzeugung eingesetzt.

Die Mittel zum Staubabscheiden können Zyklonabscheider, Staubfilter und/oder gasdichte Absperrklappen für den Gaseintritt bzw. Gasaustritt umfassen.

Die in dem (den) Zyklon(en) und auch die ggf. im (in den) vergasenden Druckreaktor(en) anfallenden Aschen können ggf. zu Aktivkohlenstoff weiter aufgearbeitet werden.

Als weitere optionale Aufbereitungsstufe zur Verbesserung der Qualität des Produktgases eignen sich Mittel zur Gasfilterung (11). Diese Mittel zur Gasfilterung (11) umfassen vorteilhafterweise eine Quenche, wobei der Gasstrom mit Wasser, das auch Reinigungsfunktion besitzt, gekühlt wird. Der größte Teil des in der Quenche eingesetzten Wasserdampfes kondensiert als Produktgas. Weiterhin kann auch eine Entschwefelung des Gasstromes erfolgen, indem etwaige Sulfoxide zu Schwefel reduziert werden. Der Schwefel kann dann als Nebenprodukt des erfindungsgemäßen Verfahrens zu anderen Zwecken eingesetzt werden.

Hierbei kommt typischerweise eine Quenche mit zweistufigem Strahlwäscher Gegenstromwäscher, Tropfenabscheider, Kreiselpumpen für den Waschwasserkreislauf und/oder Rückkühler mit Wärmeaustauscher zum Einsatz.

Weiterhin ergibt sich die Möglichkeit, die in der Quenche gegebenenfalls auftretende Kondensationswärme direkt für das erfindungsgemäße Verfahren zu nutzen. Beispielsweise kann die Kondensationswärme für den gegebenenfalls vor der Pelletierung erfolgenden, vorteilhaften Trocknungsschritt eingesetzt werden.

Das feinentstaubte und wasserstoffreiche Produktgas kann vielfältigen Einsatzmöglichkeiten zugeführt werden. Das Produktgas ist aufgrund seines hohen Heizwertes direkt als Treibstoff in einem Gasmotor oder in einer Gasturbine einsetzbar. vorteilhafterweise wird allerdings ein Reinigungsschritt vorgeschaltet, um den Kohlendioxydanteil des Produktgases zu reduzieren. Gleichfalls kann der Anteil an Wasserstoff im Produktgas durch chemische Reaktion(en) erhöht werden. So können z.B. etwaige Methanbestandteile aus dem Produktgas durch Reaktion mit Wasserdampf zu Wasserstoff und Kohlenmonoxyd umgesetzt werden. Darüber hinaus ist es möglich, durch Druckwechselabsorptionsoder Membrantrennverfahren für eine weitere Erhöhung des Wasserstoffanteils im Produktgas zu sorgen.

Dieser derart aufgereinigte Produktgasstrom mit mindestens 50% Wasserstoff, vorzugsweise mindestens 80% Wasserstoff, insbesondere bevorzugt mindestens 99% aufgereinigtem Wasserstoff kann für die Stromerzeugung in einer oder mehreren Brennstoffzelle(n) (12) eingesetzt werden.

Hierbei wird die im Wasserstoff vorliegende chemische Energie direkt in elektrische Energie umgewandelt. Der Strom kann nachfolgend entweder für den Eigenbetrieb der Vorrichtung verwendet oder aber an externe Stromabnehmer abgegeben werden. Die Brennstoffzelle(n) (12) sind vorteilhafterweise in Module aufgegliedert, die sich dann typischerweise aus 24 Zellen zusammensetzen. Die Elektroden sind vorteilerhafterweise plattenförmig ausgestaltet.

Darüber hinaus kann das wasserstoffreiche Produktgas durch Umsetzung mit Kohlenmonoxyd aus dem Abgasstrom des Heizkraftwerks zu Methanol reagieren. Hierdurch wird ein wesentlicher Beitrag zur Minimierung der Emission bei der Durchführung des erfindungsgemäßen Verfahrens geleistet. So erhält man z.B. beim Arbeiten unter Druck von 200 bis 300 atm unter Verwendung eines Katalysators, z.B. chromoxydhaltigen Zinkoxyds, bei Temperaturen von 300 bis 400°C, vorzugsweise bei ungefähr 350°C, nahezu ausschließlich Methanol als Reaktionsprodukt der Edukte Wasserstoff und Kohlenmonoxyd.

Die durch die Siebpresse, Mittel zum Entwässern (3), beispielsweise die abgepreßte wäßrige biogene Lösung (Flüssigfraktion) wird als Fermentersubstrat eingesetzt. Vorteilhafterweise wird das flüssige Fermentersubstrat mit Hilfe einer Förderpumpe und ggf. eines Plattenschiebers in einen Fermentationsreaktor (4) gepumpt. Ein solcher Fermentationsreaktor (4) hat vorteilhafterweise ein Gärvolumen von mindestens 300 m³, insbesondere bevorzugt ist ein Gärvolumen oberhalb von 500 m³. In diesem Reaktor (4) erfolgt die Vergärung der organischen Substanzen bei Temperaturen oberhalb von 35° C, vorteilhafterweise zwischen 36° C und 38° C. Bevorzugt ist (sind) diesem/n Reaktionsreaktor(en) (4) ein oder mehrere weitere Reaktionsreaktor(en) nachgeschaltet. Durch die Vergärung des Fermentersubstrats in zwei oder mehreren Reaktoren kann das Fermentationssubstrat unter verschiedenen verfahrenstechnischen Bedingungen sukzessive Vergärungsschritte durchlaufen. Insbesondere ist hierbei eine kontinuierliche Steigerung der Reaktionstemperatur in der Reihenfolge der Reaktoren möglich, weswegen ein breites Spektrum von Mikroorganismen, jeweils unter Berücksichtigung des spezifischen Temperaturoptimums, für deren jeweilige Stoffwechselvorgänge, an der Fermentation beteiligt werden kann. Insoweit kann ein weitestgehender Abbau der organischen Substanzen sichergestellt werden, und gleichzeitig ist eine kontinuierliche Durchführung der Fermentation möglich.

Typischerweise weist die erfindungsgemäße Vorrichtung zwei Reaktoren (4) auf, wobei der erste Reaktor bei einer Temperatur von 30 bis 40°C, der zweite Reaktor bei einer Temperatur oberhalb von 50° C arbeitet. Die Verweilzeiten des Fermentationssubstrats in den beiden Reaktoren können je nach den vorliegenden Gegebenheiten variiert werden.

Sofern das flüssige Fermentersubstrat 2 oder mehrere Reaktoren durchläuft, betragen sie bis zu 15 Tage, typischerweise zwischen 5 und 10 Tage, insbesondere zwischen 8 und 10 Tage. Nach Abschluß der Fermentation im letzten, z.B. im zweiten Reaktor, tritt die fermentierte Suspension über einen Ablauf aus. Vorteilhafterweise gelangt sie hierauf in einen Sammelbehälter.

Jeder Fermentationsreaktor (4) kann mindestens ein, typischerweise zwei Rührwerk(e) aufweisen. Das (die) Rührwerk(e) stellen aufgrund ihrer Mischungswirkung eine gleichmäßige Fermentation des Substrats sicher. Zudem erfolgt hierdurch eine effektive Wärmeübertragung zwischen dem Reaktorinhalt und den bevorzugt vorhandenen Heizelementen des (der) Reaktors/en. Beim Einsatz von zwei oder mehreren Rührwerken ist deren Verteilung über den gesamten Reaktorraum vorteilhaft.

Die Heizelemente des Reaktors können einen eigenen Heizkreis bilden, der bevorzugt Außenwandheizung, Verteiler, Umwälzpumpe und Meß- und Regelungselemente umfaßt. Vorteilhafterweise weist jeder Reaktor (4) einen eigenen, unabhängigen Heizkreis auf. Die Heizelemente erlauben typischerweise die Einstellung von Temperaturen zwischen 20 und 80°C. Wärmeverluste der Reaktoren (4) können durch Ummantelung mit Isolationsschichten aus Mineralwolle weitestgehend unterbunden werden.

Jeder Fermentationsreaktor (4) weist einen Gasraum auf, in dem sich die gasförmigen Endprodukte der Fermentation von flüssigem Fermentersubstrat ansammeln. Das auf diesem Wege produzierte Biogas wird aus dem Gasraum abgezogen und dann bestimmungsgemäß weiterverwendet. Die Temperaturen und Drücke im Reaktor (4) werden laufend kontrolliert. In Form eines Regelkreises kann dann die Beschikkung des Reaktors mit abgepreßter Lösung und/oder der Abzug des Biogases aus dem Reaktorgasraum entsprechend reguliert werden. Der Reaktor (4) kann vorteilhafterweise eine Überdrucksicherung aufweisen, um eine sichere Betriebsführung des erfindungsgemäßen Verfahrens zu gestatten.

In einer besonders vorteilhaften Ausführungungsform umfaßt die erfindungsgemäße Vorrichtung zudem einen oder mehrere Mischbehälter. In diesem Fall kann die wäßrige biogene Substratlösung vor der Beschickung des ersten Reaktors mit Feststoffen versetzt werden. Feststoff und Fermentersubstrat werden zunächst, z.B. durch ein oder mehrere Rührwerke, zerkleinert und hierdurch vermischt. Sobald der Feststoff gelöst vorliegt, fördert eine Pumpe die Suspension in den (die) Reaktor(en) (4). Die Beschikkung von Reaktoren (4) kann demnach über die Zufuhr von abgepreßter biogener wäßriger Lösung, nach Mischung der Flüssigfraktion mit Feststoff oder aber als Kombination der beiden vorgenannten Beschickungsarten erfolgen.

Weitere optionale Elemente des (der) Reaktor(en) (4) in einer erfindungsgemäßen Vorrichtung ist (sind) ein oder mehrere Absaugpumpe(n), die im oberen oder im unteren Bereich des (der) Reaktors/en vorgesehen sein kann (können). Diese Absaugpumpen erlauben die Austragung von Schwerstoffen am Boden der Reaktoren bzw. die Austragung etwaiger Leichtstoffe im oberen Bereich der Reaktoren. Typischerweise gestattet eine Öffnung in jeden Reaktor Wartungs- und Reparaturarbeiten.

Die pro Jahr erzeugte Biogasmenge beträgt beispielsweise zwischen 500.000 und 1.000.000 m³, abhängig von der Beschickung, den Laufzeiten und der Größe bzw. der Zahl der Reaktoren.

Das aus dem oberen Bereich des (der) Fermentationsreaktors/en (4) abgezogene Biogas kann einem Heizkraftwerk (10) und/oder einem Druckreaktor (5) der erf indungsgemäβen Vorrichtung zugeführt werden. Vorteilhafterweise erfolgt nach dem Abzug des Biogases aus den Reaktoren zunächst eine Kondensatabscheidung durch Kühlung mit Wasser und danach eine Entschwefelung durch Oxidation des im Biogas enthaltenen Schwefelwasserstoffs durch stöchiometrische Luftzugabe. Daraufhin kann das Biogas zunächst in einem oder mehreren Gasspeichern zwischengespeichert werden. Diese Gasspeicher sind typischerweise als Folienspeicher mit Außenbehälter ausgelegt.

Das ggf. getrocknete und entschwefelte Biogas wird vorteilhafterweise zum größten Teil einem Heizkraftwerk (10) zugeführt. In diesem Heizkraftwerk (10) wird die im Biogas gespeicherte chemische Energie durch Verbrennung desselben freigesetzt. Diese chemische Energie kann zur Stromerzeugung, z.B. zur Stromerzeugung für den Betrieb einer erfindungsgemäßen Vorrichtung, genutzt werden. Besonders geeignet für die Umwandlung der chemischen Energie in Strom sind Blockheizkraftwerke. Diese werden typischerweise mit Gasmotoren und/oder Gasturbinen betrieben, die mit einem Verstromungsaggregat und einem Wärmetauscher ausgestattet sein können. Der Wärmeaustauscher kann auch dazu herangezogen werden, den Wärmebedarf für die Temperierung des (der) Reaktor(en) zu decken.

Der in dem (den) Fermentationsreaktor(en) (4) vergorene flüssige Rückstand bzw. die vergorene Suspension kann durch eine Presse entwässert werden. Der dabei erhaltene flüssige Rückstand kann dann als Flüssigdünger für die Landwirtschaft eingesetzt werden. Gleichfalls kann der nach dem Pressen verbliebene feste Rückstand als Dünger aufbereitet werden.

## Patentansprüche

1. Verfahren zur Aufbereitung von Reststoffgemengen und zur Konversion von kohlenstoffhaltigen Rest- oder Rohstoffen in den Restsoffgemengen, bei dem
(a) die in den Reststoffgemengen enthaltenen kohlenstoffhaltigen Rest- oder Rohstoffe von anorganischen und/oder metallischen Materialien getrennt werden;
(c) Flüssigrohmaterial der Reststoffgemenge in einem Reaktor einer Fermentation unterzogen wird; und
(d1 ) das im Zuge der Fermentation gebildete Biogas in mindestens einem Kraftwerk zur Energiegewinnung eingesetzt wird,
**dadurch gekennzeichnet, daß**
(b) die kohlenstoffhaltigen Rest- und Rohstoffe nach dem Schritt (a) entwässert und pelletiert werden;
(c) das im Zuge der Entwässerung anfallende Abpreßwasser in einem oder mehreren Reaktoren der Fermentation unterzogen wird;
(d2) das im Zuge der Fermentation (c) gebildete Biogas auch/oder mindestens einem Druckgasreaktor zugeführt wird, in dem die pelletierten Rest- oder Rohstoffe aufgeschlossen und vergast werden.

2. Verfahren nach Anspruch 1, **dadurch gekennzeichnet, daß** die gemäß (a) von den metallischen oder anorganischen Feststoffen getrennten kohlenstoffhaltigen Rest- oder Rohstoffe zerkleinert und/oder gesiebt werden.

3. Verfahren nach Anspruch 1 oder 2, **dadurch gekennzeichnet, daß** der Wasseranteil in den gemäß (b) pelletierten Rest- oder Rohstoffen vor der Zufuhr in einen Druckreaktor auf höchstens 25 Gew.-% vermindert wird.

4. Verfahren nach einem der vorgenannten Ansprüche, **dadurch gekennzeichnet, daß** die nicht-gasförmigen Fermentierungs-produkte gemäß (c) in eine flüssige und eine feste Phase aufgetrennt werden.

5. Verfahren nach Anspruch 4, **dadurch gekennzeichnet, daß** die flüssige Phase durch eine Entwässerungspresse von der festen Phase abgeschieden wird.

6. Verfahren nach einem der vorgenannten Ansprüche, **dadurch gekennzeichnet, daß** die Aufschlußreaktion (d2) im Druckreaktor bei einer Temperatur zwischen 550 °C und 850 °C durchgeführt wird.

7. Verfahren nach einem der vorgenannten Ansprüche, **dadurch gekennzeichnet, daß** die Aufschlußreaktion (d2) als Wasserdampfdruckreaktion erfolgt.

8. Verfahren nach einem der vorgenannten Ansprüche, **dadurch gekennzeichnet, daß** von den bei der Aufschlußreaktion (d2) im Druckreaktor gebildeten, gasförmigen Bestandteilen Wasserstoff abgetrennt wird.

9. Verfahren nach Anspruch 8, **dadurch gekennzeichnet, daß** die Auftrennung durch einen Gasfilter erfolgt.

10. Verfahren nach Anspruch 8 oder 9, **dadurch gekennzeichnet, daß** der abgetrennte Wasserstoff mit Abgasstrom, insbesondere kohlenmonoxidhaltigen Abgasen, zu Methanol umgesetzt wird.

11. Verfahren nach Anspruch 8, **dadurch gekennzeichnet, daß** der bei der Energiegewinnung aus Biogas (d1) im Kraftwerk gebildete Abgasstrom zur Methanolsynthese eingesetzt wird.

12. Verfahren nach Anspruch 8 oder 9, **dadurch gekennzeichnet, daß** die nach Abtrennung des Wasserstoffs verbliebenen, gasförmigen Bestandteile dem Kraftwerk zugeführt und zur Energiegewinnung eingesetzt werden.

13. Vorrichtung zur Aufbereitung von Reststoffgemengen und zur Konversion von kohlenstoffhaltigen Restoder Rohstoffen in den Reststoffgemengen zur Durchführung eines Verfahrens nach einem der Ansprüche 1 bis 12, **dadurch gekennzeichnet, daß** sie mindestens einen Fermentierungsreaktor, mindestens ein Kraftwerk und mindestens einen Druckreaktor aufweist.

14. Vorrichtung nach Anspruch 13, **dadurch gekennzeichnet, daß** der (die) Druckreaktor(en) als Wasserdampfdruckreaktor(en) ausgestaltet ist (sind).

15. Vorrichtung nach Anspruch 13 oder 14, **dadurch gekennzeichnet, daß** das (die) Kraftwerk(e) als Blockheizkraftwerk(e) mit Gasmotor- und/oder Gasturbinenbetrieb ausgestaltet ist (sind).

## Claims

1. Process for preparing residual material mixtures and for converting carbon-containing residual or raw materials in the residual material mixtures, in which
(a) the carbon-containing residual or raw materials present in the residual material mixtures are separated from inorganic and/or metallic materials;
(c) liquid raw material of the residual material mixture is subjected to fermentation in a reactor, and
(d1) the biogas formed in the course of fermentation is used for energy production in at least one power plant,
**characterised in that**
(b) the carbon-containing residual and raw materials are dewatered after step (a) and pelleted;
(c) the expelled water occurring in the course of dewatering is subjected to fermentation in one or more reactors;
(d2) the biogas formed in the course of fermentation (c) is supplied also/or to at least one pressurised gas reactor, in which the pelleted residual or raw materials are digested and vaporised.

2. Process according to claim 1, **characterised in that** the carbon-containing residual or raw materials separated according to (a) from the metallic or inorganic solids are comminuted and/or screened.

3. Process according to claim 1 or 2, **characterised in that** the water portion in the residual or raw materials pelleted according to (b) is reduced to 25 wt.% at the most before supplying to a pressure reactor.

4. Process according to one of the aforementioned claims, **characterised in that** the non-gaseous fermentation products according to (c) are separated into a liquid and a solid phase.

5. Process according to claim 4, **characterised in that** the liquid phase is separated off from the solid phase by a dewatering press.

6. Process according to one of the aforementioned claims, **characterised in that** the digestion reaction (d2) is carried out in the pressure reactor at a temperature between 550°C and 850°C.

7. Process according to one of the aforementioned claims, **characterised in that** the digestion reaction (d2) takes place as a steam pressure reaction.

8. Process according to one of the aforementioned claims, **characterised in that** hydrogen is separated off from the gaseous constituents formed in the digestion reaction (d2) in the pressure reactor.

9. Process according to claim 8, **characterised in that** separation takes place through a gas filter.

10. Process according to claim 8 or 9, **characterised in that** the hydrogen separated off is reacted with waste gas stream, in particular waste gases containing carbon monoxide, to give methanol.

11. Process according to claim 8, **characterised in that** the waste gas stream formed during energy production from biogas (d1) in the power plant is used for methanol synthesis.

12. Process according to claim 8 or 9, **characterised in that** the gaseous constituents remaining after separation of the hydrogen are supplied to the power plant and used for energy production.

13. Device for preparing residual material mixtures and for converting carbon-containing residual or raw materials in the residual material mixtures for carrying out a process according to one of claims 1 to 12, **characterised in that** it has at least one fermentation reactor, at least one power plant and at least one pressure reactor.

14. Device according to claim 13, **characterised in that** the pressure reactor(s) is (are) designed as steam pressure reactor(s).

15. Device according to claim 13 or 14, **characterised in that** the power plant(s) is (are) designed as block thermoelectric power plant(s) with gas engine and/or gas turbine operation

## Revendications

1. Procédé pour traiter des mélanges de matières résiduelles et pour la conversion de matières carbonées résiduelles ou brutes dans les mélanges de matières résiduelles, dans lequel :
(a) les matières carbonées résiduelles ou brutes présentes dans les mélanges de matières résiduelles sont séparées des matériaux inorganiques et/ou métalliques ;
(c) la matière brute liquide du mélange de matières résiduelles est soumise à une fermentation dans un réacteur, et
(d1) le biogaz formé au cours de la fermentation est mis en oeuvre dans au moins une usine génératrice pour la production d'énergie,
**caractérisé en ce que**
(b) les matières carbonées résiduelles ou brutes carbonées selon l'étape (a) sont déshydratées et transformées en pellets ;
(c) l'eau extraite produite au cours de la déshydratation est soumise à une fermentation dans un ou plusieurs réacteurs ;
(d2) le biogaz formé au cours de la fermentation (c) est amené dans au moins un réacteur à gaz comprimé, dans lequel les matières résiduelles ou brutes en pellets sont digérées et gazéifiées.

2. Procédé selon la revendication 1, **caractérisé en ce que** les matières carbonées résiduelles ou brutes, séparées selon (a) des substances solides métalliques ou inorganiques, sont concassées et/ou tamisées.

3. Procédé selon la revendication 1 ou 2, **caractérisé en ce que** la quantité d'eau dans les matières résiduelles ou brutes en pellets selon (b) est diminuée avant l'apport dans un réacteur sous pression de jusqu'à 25% en poids.

4. Procédé selon l'une quelconque des revendications précédentes, **caractérisé en ce que** le produit de fermentation non gazeux selon (c) est séparé en une phase liquide et en une phase solide.

5. Procédé selon la revendication 4, **caractérisé en ce que** la phase liquide est extraite de la phase solide par une presse d'épuisement.

6. Procédé selon l'une quelconque des revendications précédentes, **caractérisé en ce que** la réaction de digestion (d2) est réalisée dans le réacteur sous pression à une température allant de 550°C à 850°C.

7. Procédé selon l'une quelconque des revendications précédentes, **caractérisé en ce que** la réaction de digestion (d2) est réalisée comme une réaction sous pression de vapeur d'eau.

8. Procédé selon l'une quelconque des revendications précédentes, **caractérisé en ce que** l'on sépare l'hydrogène des constituants gazeux, formés dans le réacteur sous pression lors de la réaction de digestion (d2).

9. Procédé selon la revendication 8, **caractérisé en ce que** la séparation est réalisée par un filtre à gaz.

10. Procédé selon la revendication 8 ou 9, **caractérisé en ce que** l'hydrogène séparé est transformé en méthanol avec un courant de gaz résiduaires, en particulier de gaz résiduaires contenant du monoxyde de carbone.

11. Procédé selon la revendication 8, **caractérisé en ce que** le courant de gaz résiduaire formé dans l'usine génératrice lors de la production d'énergie à partir du biogaz (d1) est mis en oeuvre pour la synthèse de méthanol.

12. Procédé selon la revendication 8 ou 9, **caractérisé en ce que** les constituants gazeux, restant après la séparation de l'hydrogène, sont envoyés dans l'usine génératrice et mis en oeuvre pour la production d'énergie.

13. Dispositif de traitement de mélanges de matières résiduelles et de conversion de matières carbonées résiduelles ou brutes dans les mélanges de matières résiduelles, pour réaliser un procédé selon l'une quelconque des revendications 1 à 12, **caractérisé en ce qu'**il présente au moins un réacteur de fermentation, au moins une usine énergétique et au moins un réacteur sous pression.

14. Dispositif selon la revendication 13, **caractérisé en ce que** le ou les réacteurs sous pression sont équipés comme des réacteurs sous pression de vapeur d'eau.

15. Dispositif selon la revendication 13 ou 14, **caractérisé en ce que** la ou les usines génératrices sont équipées comme des centrales thermiques avec entraînement à moteur à gaz et/ou turbines à gaz.
